# EUROPÄISCHE PATENTANMELDUNG

(11) **EP 2 229 953 A1**
(43) Veröffentlichungstag der Anmeldung: **22.09.2010**
(21) Anmeldenummer: 10000937.2
(22) Anmeldetag: 28.07.2004
(51) Int. Cl.: A61K 39/00, A61K 39/145

(54) **Transfektion von Blutzellen mit mRNA zur Immunstimulation und Gentherapie**

(30) Priorität: 05.08.2003 DE 10335833
(62) Teilanmeldung aus: 07020335.1
(71) Anmelder: CureVac GmbH, 72076 Tübingen (DE)
(72) Erfinder: Hoerr, Ingmar, Dr., 72070 Tübingen (DE); Von der Mülbe, Florian, 70563 Stuttgart (DE); Pascolo, Steve, Dr., 8004 Zürich (CH)
(74) Vertreter: Graf von Stosch, Andreas

(57) **Zusammenfassung**

Die vorliegende Erfindung betrifft eine pharmazeutische Zusammensetzung, die Blutzellen bzw. hämatopoietische Zellen, bspw. rote Blutzellen (Erythrocyten), Granulocyten, mononucleäre Zellen (PBMCs) und/oder Blutplättchen, in Verbindung mit einem pharmazeutisch verträglichen Träger und/oder Vehikel enthält, wobei die Zellen mit mindestens einer mRNA transfiziert sind, die mindestens einen für mindestens ein Antigen codierenden Bereich umfaßt Weiterhin wird erfindungsgemäß ein Verfahren zur Herstellung der vorgenannten pharmazeutischen Zusammensetzung sowie die Verwendung derartig transfizierter Blutzellen zur Herstellung von Arzneimitteln bzw. pharmazeutischen Zusammensetzungen zur Immunstimulation gegen die von der mRNA codierten Antigene offenbart. Die erfindungsgemäßen Gegenstände dienen insbesondere der Therapie von und/oder Prophylaxe gegen Krebs oder Infektionskrankheiten und sind darüber hinaus in der Gentherapie einsetzbar.

SEQUENCE LISTING

<110> CureVac GmbH

<120> Transfektion von Blutzellen mit mRNA zur Immunstimulation und
Gentherapie

<130> CU01P019WOEPT1T1

<160> 3

<170> PatentIn version 3.3

<210> 1
<211> 13
<212> DNA
<213> Artificial

<220>
<223> Beschreibung der Sequenz: Kozak-Sequenz (s. Beschreibung S. 31)

<400> 1
gccgccacca ugg 13

<210> 2
<211> 15
<212> DNA
<213> Artificial

<220>
<223> Beschreibung der Sequenz: generische Sequenz einer
Stabilisierungssequenz (s. Beschreibung S. 32)

<220>
<221> variation
<222> (1)..(1)
<223> /replace="cytosine"

/replace="uracile"

<220>
<221> variation
<222> (5)..(5)
<223> /replace="cytosine"

/replace="uracile"

/replace="guanosine"

/replace="adenosine", or any othe nucleic acid

<220>
<221> repeat_unit
<222> (5)..(5)
<223> x = any number

<220>
<221> variation
<222> (9)..(9)
<223> /replace="uracile"

/replace="adenosine"

<220>
<221>  repeat_unit
<222>  (10)..(10)
<223>  x = any number

<220>
<221>  variation
<222>  (10)..(10)
<223>  /replace="pyrimidine"

<220>
<221>  variation
<222>  (13)..(13)
<223>  /replace="cytosine"

       /replace="uracile"

<400>  2
nccancccnn ucncc                                                    15

<210>  3
<211>  7
<212>  DNA
<213>  Artificial

<220>
<223>  Sequenz aus 3'-UTR-Segment des fuer den Transferrin-Rezeptor
       kodierenden Gensn (s. S. 9)

<400>  3
gaacaag                                                              7

## Beschreibung

Die vorliegende Erfindung betrifft eine pharmazeutische Zusammensetzung, die Blutzellen bzw. hämatopoietische Zellen, bspw. rote Blutzellen (Erythrocyten), Granulocyten, mononucleäre Zellen (PBMCs) und/oder Blutplättchen, in Verbindung mit einem pharmazeutisch verträglichen Träger und/oder Vehikel enthält, wobei die Zellen mit mindestens einer mRNA transfiziert sind, die mindestens einen für mindestens ein Antigen codierenden Bereich umfaßt. Weiterhin wird erfindungsgemäß ein Verfahren zur Herstellung der vorgenannten pharmazeutischen Zusammensetzung sowie die Verwendung derartig transfizierter Blutzellen zur Herstellung von Arzneimitteln bzw. pharmazeutischen Zusammensetzungen zur Immunstimulation gegen die von der mRNA codierten Antigene offenbart. Die erfindungsgemäßen Gegenstände dienen insbesondere der Therapie von und/oder Prophylaxe gegen Krebs oder Infektionskrankheiten und sind darüber hinaus in der Gentherapie einsetzbar.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide. Grundsätzlich vorteilhafte Verfahren beruhen dabei auf der direkten oder indirekten (über Transfektion geeigneter Zellen) Einbringung von für das jeweilige Protein codierender (m)RNA.

Derzeit wird bei der Verwendung von mRNA zur Vakzinierung, insbesondere zur Vakzinierung gegen Tumorantigene, die für das entsprechende Antigen codierende mRNA üblicherweise in (professionelle) antigen-präsentierende Zellen (engl. "antigen presenting cell", APC), insbesondere dendritische Zellen (DCs) *in vitro* transfiziert (Boczkowski et al. (1996) J. Exp. Med. 184 (2): 465 - 472; WO 97/41210). Die DCs werden aus dem Blut des Patienten gewonnen, indem unter Verwendung eines speziellen Cytokin-Cocktails entsprechende Monocyten in DCs differenziert werden. Dieses Verfahren ist jedoch langwierig. Insbesondere vergehen zwischen Blutentnahme und Transfektion üblicherweise etwa 7 Tage (d.h. etwa 1 Woche *In vitro*-Kultur zur Differenzierung der DCs), so dass es im allgemeinen 9 Tage (2 Tage Reifung der DCs nach der Transfektion) dauert, bis die Zellen in den Patienten rückinjiziert werden. Entscheidend für diese lange Zeitspanne ist dabei die Dauer der *In vitro* -Kultur zur Differenzierung der DCs zwischen Blutentnahme und Transfektion. Darüber hinaus sind bei der Erzeugung von DCs GMP (engl. "good manufacturing practice")-Bedingungen einzuhalten. Daher ist dieses Verfahren extrem kostspielig, wobei auch zu beachten ist, daß der Patient während der DC-Produktion gegebenenfalls gesondert unterzubringen ist.

Der vorliegenden Erfindung liegt daher die Aufgabe zugrunde, ein neuartiges System der Immunstimulation von Patienten gegen spezifische Antigene, insbesondere solche aus Tumoren und infektiösen Keimen, bereitzustellen, das die Nachteile von im Stand der Technik bekannten Verfahren überwindet, insbesondere eine aufwendige und teure Erzeugung von APCs *in vitro* vermeidet.

Diese Aufgabe wird durch die in den Ansprüchen gekennzeichneten Ausführungsformen der vorliegenden Erfindung gelöst.

Insbesondere wird erfindungsgemäß eine pharmazeutische Zusammensetzung bereitgestellt, die mit mindestens einer mRNA transfizierte Blutzellen bzw. hämatopoietische Zellen, bspw. rote Blutzellen (Erythrocyten), Granulocyten, mononucleäre Zellen (engl. "peripherial blood mononuclear cells", PBMCs) und/oder Blutplättchen (Thrombocyten), in Verbindung mit einem pharmazeutisch verträglichen Träger und/oder Vehikel enthält, wobei die mindestens eine mRNA mindestens einen für mindestens ein Antigen codierenden Bereich umfaßt.

Die vorliegende Erfindung beruht auf der überraschenden Erkenntnis, daß es zur Vakzinierung von Patienten gegenüber bestimmten Antigenen, die von der erfindungsgemäßen mRNA codiert werden, nicht erforderlich ist, bspw. aus dem Blut eines Individuums, insbesondere des zu behandelnden Patienten selbst, gewonnene Blutzellen, bspw. PBMCs, mittels aufwendiger, langer und teurer Zellkulturtechniken in eine Population von Zellen mit hohem Anteil an professionellen Antigen präsentierenden Zellen (engl. "antigen presenting cells", APCs), insbesondere dentritischen Zellen (DCs), zu differenzieren, sondern es für eine erfolgreiche Immunstimulation ausreicht, Blutzellen direkt mit der für ein oder mehrere Antigene codierenden mRNA zu transfizieren, um so eine pharmazeutische Zusammensetzung zu erhalten, die bspw. in dem Patienten selbst, von dem die Blutzellen, insbesondere die vorstehend genannten Teilpopulationen davon, gewonnen wurden, eine geeignete Immunstimulation bewirkt, die sich vorzugsweise gegen ein oder mehrere Antigen(e) aus einem Tumor oder gegen ein oder mehrere Antigen(e) eines pathogenen Keims bzw. Agens richtet.

Die Blutzellen, insbesondere die vorstehend genannten Teilpopulationen davon, in der erfindungsgemäßen pharmazeutischen Zusammensetzung sind besonders **dadurch gekennzeichnet, daß** sie einen geringen Anteil an ausdifferenzierten professionellen APCs, wie DCs, enthalten. Vorzugsweise enthalten die transfizierten Zellen weniger als 5 %, mehr bevorzugt nicht mehr als 2 % DCs, wenn sie in der pharmazeutischen Zusammensetzung der vorliegenden Erfindung enthalten sind. Unter "Blutzellen", wird erfindungsgemäß daher bevorzugt ein Gemisch oder eine angereichte bis im wesentlichen reine Population von roten Blutzellen, Granulocyten, mononucleären Zellen (PBMCs) und/oder Blutplättchen, aus Vollblut, Blutserum oder einer anderen Quelle, bspw. aus der Milz oder Lymphknoten verstanden, wobei nur ein geringer Anteil an professionellen APCs vorhanden ist. Vorzugsweise handelt es sich bei den Blutzellen der vorliegenden Erfindung - im Gegensatz zum Stand der Technik - um frische Blutzellen, d.h. es vergeht zwischen Gewinnung der Blutzellen (insbesondere der Blutentnahme) und der Transfektion nur eine kurze Zeit, bspw. weniger als 12 h, vorzugsweise weniger als 6 h, mehr bevorzugt weniger als 2 h, besonders bevorzugt weniger als 1 h.

Wie bereits vorstehend erwähnt, werden für die erfindungsgemäße pharmazeutische Zusammensetzung vorzugsweise solche Blutzellen verwendet, die aus demjenigen Patienten selbst stammen, der mit der pharmazeutischen Zusammensetzung der vorliegenden Erfindung behandelt wird. Daher enthält die erfindungsgemäße pharmazeutische Zusammensetzung vorzugsweise autologe Blutzellen.

Gemäß einer weiteren bevorzugten Ausführungsform der vorliegenden Erfindung umfaßt die zur Transfektion der erfindungsgemäßen Zellen verwendete mRNA einen Bereich, der für mindestens ein Antigen aus einem Tumor oder eines pathogenen Agens bzw. eines pathogenen Keims codiert.

Der Begriff "Antigen aus einem Tumor" bedeutet erfindungsgemäß, dass das entsprechende Antigen in mit einem Tumor assozierten Zellen exprimiert wird. Daher sind erfindungsgemäß Antigene aus Tumoren insbesondere solche, die in den entarteten Zellen selbst produziert werden. Vorzugsweise handelt es sich dabei um auf der Oberfläche der Zellen lokalisierte Antigene. Des Weiteren sind die Antigene aus Tumoren aber auch solche, die in Zellen exprimiert werden, welche nicht selbst (oder ursprünglich nicht selbst) entartet sind (waren), jedoch mit dem in Rede stehenden Tumor assoziiert sind. Dazu gehören bspw. auch Antigene, die mit Tumor-versorgenden Gefäßen bzw. deren (Neu-)Bildung zusammenhängen, insbesondere solche Antigene, die mit der Neovaskularisierung oder Angiogenese assoziiert sind, bspw. Wachstumsfaktoren wie VEGF, bFGF, usw. Weiterhin umfassen derartige mit einem Tumor zusammenhängende Antigene solche aus Zellen des den Tumor einbettenden Gewebes. Zu nennen sind hier entsprechende Antigene von Bindegewebszellen, z.B. Antigene der extrazellulären Matrix. Zur erfindungsgemäßen Immunstimulation können die mRNA-Moleküle auch eine cDNA-Bibliothek aus einem Tumorgewebe repräsentieren. Es kann sich dabei auch um einen Teil einer entsprechenden Bibliothek handeln, vorzugsweise um denjenigen Teil der cDNA-Bibliothek der für die tumorspezifischen Antigene codiert. Entsprechende Herstellungsverfahren hierzu werden nachstehend im Zusammenhang mit dem erfindungsgemäßen Verfahren zur Herstellung der pharmazeutischen Zusammensetzung ausführlich dargelegt.

Erfindungsgemäß wird die pharmazeutischen Zusammensetzung, enthaltend Blutzellen, bspw. PBMCs, rote Blutzellen, Granulocyten und/oder Blutplättchen, die mit einer (oder mehreren) mRNAs transfiziert sind, zur Therapie bzw. Impfung, d.h. Vakzinierung, insbesondere zur Behandlung oder Prävention (Prophylaxe) von Krebserkrankungen verwendet. Die Vakzinierung beruht auf der Einbringung eins Antigens (oder mehrerer Antigene) eines Tumors, im vorliegenden Fall der genetischen Information für das Antigen in Form der für das oder die Antigen(e) codierenden mRNA, in die Blutzellen. Die in der pharmazeutischen Zusammensetzung enthaltene mRNA wird in den Zellen in das (Tumor-)Antigen translatiert, d.h. das von der modifizierten mRNA codierte Polypeptid bzw. antigene Peptid wird exprimiert, wodurch nach Einbringung der pharmazeutischen Zusammensetzung, enthaltend die transfizierten Zellen, eine gegen dieses Polypeptid bzw. antigene Peptid gerichtete Immunantwort stimuliert wird. Im vorliegenden Fall der Verwendung als genetische Vakzine zur Behandlung von Krebs wird daher die Immunantwort durch Einbringung der genetischen Information für Antigene aus einem Tumor, insbesondere Proteine, die ausschließlich auf Krebszellen exprimiert werden, erreicht, in dem eine erfindungsgemäße pharmazeutische Zusammensetzung verabreicht wird, die Blutzellen, z.B. rote Blutzellen, Granulocyten, PBMCs und/oder Blutplättchen, enthält, welche mit einer für ein derartiges Krebsantigen codierenden mRNA transfiziert sind. Dadurch wird das oder die Krebsantigen(e) im Organismus Immunzellen präsentiert, wodurch eine Immunantwort hervorgerufen wird, die wirksam gegen die Krebszellen gerichtet ist.

Bei der Vakzinierung gegen einen pathogenen Keim, wie ein Virus, ein Bakterium oder ein protozoologischer Keim, wird daher vorzugsweise ein Oberflächenantigen eines derartigen Keims zur Vakzinierung mit Hilfe der erfindungsgemäßen pharmazeutischen Zusammensetzung, enthaltend mit der für das Oberflächenantigen codierende mRNA transfizierte Blutzellen verwendet. Daher wird die erfindungsgemäße pharmazeutische Zusammensetzung auch insbesondere gegen Infektionserkrankungen (z.B. virale Infektionskrankheiten, wie AIDS (HIV), Hepatitis A, B oder C, Herpes, Herpes Zoster (Varizellen), Röteln (Rubeola-Virus), Gelbfieber, Dengue usw. (Flavi-Viren), Grippe (influenza-Viren), hämorrhagische Infektionskrankheiten (Marburg- oder Ebola-Viren), bakterielle Infektionserkrankungen, wie Legionärskrankheit (Legionella), Magengeschwür (Helicobacter), Cholera (Vibrio), *E*.*coli*-Infektionen, Staphylokokken-Infektionen, Salmonellen-Infektionen oder Streptokokken-Infektionen (Tetanus), oder protozoologische Infektionserkrankungen (Malaria, Schlafkrankheit, Leishmaniose, Toxoplasmose, d.h. Infektionen durch Plasmodium, Trypanosomen, Leishmania und Toxoplasmen, sowie Chlamydien-Infektionen, bspw. mit Chlamydia pneumoniae oder Chlamydia trachomatis) eingesetzt. Vorzugsweise werden auch im Falle von Infektionserkrankungen die entsprechenden Oberflächenantigene mit dem stärksten antigenen Potenzial durch die mRNA codiert. Bei den genannten Genen pathogener Keime bzw. Organismen, insbesondere bei viralen Genen, ist dies typischerweise eine sekretierte Form eines Oberflächenantigens.

Im Falle der Immunstimulation gegen einen pathogenen Keim können die transfizierten mRNA-Moleküle ebenfalls eine cDNA-Bibliothek oder einen Teil davon bestimmter Zelltypen umfassen. In diesem Fall werden erfindungsgemäß bevorzugt cDNA-(Teil)-Bibliotheken von mit dem pathogenen Agens infizierten Zellen verwendet. So kann bspw. im Falle einer HIV-Infektion eine mRNA-Population aus mit HIV infizierten, vorzugsweise autologen, T-Zellen verwendet werden, um eine Immunstimulation gegen die Produkte der durch das pathogene Agens, im genannten Beispiel HIV, hochregulierten Wirtsgene zu erzielen.

Des weiteren werden erfindungsgemäß bevorzugt für Polypeptide codierende mRNAs eingesetzt, wobei es sich bei den Polypeptiden um Polyepitope, bspw. der vorstehend genannten Antigene, insbesondere Oberflächenantigene von pathogenen Keimen bzw. Organismen oder Tumorzellen, vorzugsweise sekretierter Proteinformen, handelt.

In ihrer Verwendung als Vakzine kommt die erfindungsgemäße pharmazeutische Zusammensetzung insbesondere zur Behandlung von Krebserkrankungen (wobei die mRNA vorzugsweise für ein tumorspezifisches Oberflächenantigen (TSSA) codiert), bspw. zur Behandlung von malignem Melanom, Kolon-Karzinom, Lymphomen, Sarkomen, kleinzelligem Lungenkarzinom, Blastomen usw., in Betracht. Spezifische Beispiele von Tumorantigenen sind u.a. 707-AP, AFP, ART-4, BAGE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pml/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1.

Gemäß einer weiteren bevorzugten Ausführungsform ist das oder sind die Antigen(e) aus einem Tumor oder eines pathogenen Agens ein Polyepitop des/der Antigens/Antigene aus einem Tumor oder eines pathogenen Agens. Ein "Polyepitop" eines Antigens bzw. mehrerer Antigene ist eine Aminosäuresequenz, in der mehrere oder viele Regionen des/der Antigens/Antigene repräsentiert sind, die mit dem Antigen-bindenden Teil eines Antikörpers oder mit einem T-Zell-Rezeptor in Wechselwirkung treten. Das Polyepitop kann dabei vollständig und unmodifiziert vorliegen. Es kann jedoch gemäß der vorliegenden Erfindung, insbesondere zur Optimierung der Antikörper/Antigen- bzw. T-Zell-Rezeptor/Antigen-Wechselwirkung, auch modifiziert vorliegen. Eine Modifikation gegenüber dem Wildtyp-Polyepitop kann bspw. eine Deletion, Addition und/oder Substitution eines oder mehrerer Aminosäurereste umfassen. Dementsprechend wird/werden in der für das modifizierte Polyepitop codierenden mRNA der vorliegenden Erfindung gegenüber der für das Wildtyp-Polyepitop codierenden mRNA ein oder mehrere Nucleotide entfernt, hinzugefügt und/oder ersetzt.

Darüber hinaus kann die erfindungsgemäße pharmazeutische Zusammensetzung im Rahmen der Gentherapie eingesetzt werden, wobei die RNA bspw. für ein in den Blut- oder hämatopoietischen Zellen eines Patienten fehlendes oder dort nicht funktionsfähiges Protein, z.B. ein Enzym, codiert.

Um die Stabilität und die Transfektionseffizienz der (m)RNA zu erhöhen, weist vorzugsweise jede in die Blutzellen der vorliegenden Erfindung einzubringende (m)RNA eine oder mehrere Modifikationen, insbesondere chemische Modifikationen, auf, welche den Transfer der (m)RNA (eine oder mehrere) in die zu transfizierenden Zellen verbessern und/oder die Expression des/der codierten Antigens/Antigene erhöht/erhöhen.

Beispielsweise gibt es in den Sequenzen eukaryotischer mRNAs destabilisierende Sequenzelemente (DSE), an welche Signalproteine binden und den enzymatischen Abbau der mRNA *in vivo* regulieren. Daher werden zur weiteren Stabilisierung der mRNA gegebenenfalls im für das mindestens eine Antigen aus einem Tumor oder eines pathogenen Agens codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es erfindungsgemäß ebenfalls bevorzugt, gegebenenfalls in den nicht-translatierten Bereichen (3'- und/oder 5'-UTR) vorhandene DSE aus der mRNA zu eliminieren.

Derartige destabilisierende Sequenzen sind bspw. AU-reiche Sequenzen ("AU-RES"), die in 3'-UTR-Abschnitten zahlreicher instabiler mRNA vorkommen (Caput et al., Proc. Natl. Acad. Sci. USA 1986, 83: 1670 bis 1674). Die in der vorliegenden Erfindung verwendeten RNA-Moleküle sind daher vorzugsweise derart gegenüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Auch diese Sequenzmotive werden bevorzugt in der modifizierten mRNA, die zur Transfektion der Blutzellen verwendet wird, eliminiert.

Einem Fachmann sind verschiedene Verfahren geläufig, die zur Substitution von Codons in der erfindungsgemäß modifizierten mRNA geeignet sind. Im Falle kürzerer codierender Bereiche (die für biologisch wirksame oder antigene Peptide codieren) kann bspw. die gesamte mRNA chemisch unter Verwendung von Standardtechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt (Maniatis et al., Molecular Cloning: A Laboratory Manual, Cold Spring Harbor Laboratory Press, 3. Aufl., Cold Spring Harbor, NY, 2001).

Bei diesem Verfahren wird zur Herstellung der mRNA daher ein entsprechendes DNA-Molekül *in vitro* transkribiert. Diese DNA-Matrize besitzt einen geeigneten Promotor, bspw. einen T7- oder SP6-Promotor, für die *In vitro*-Transkription, dem die gewünschte Nucleotidsequenz für die herzustellende mRNA und ein Terminationssignal für die in *In vitro* -Transkription folgen. Erfindungsgemäß wird das DNA-Molekül, das die Matrize des herzustellenden RNA-Konstrukts bildet, üblicherweise durch fermentative Vermehrung und anschließende Isolierung als Teil eines in Bakterien replizierbaren Plasmids hergestellt. Als für die vorliegende Erfindung geeignete Plasmide können bspw. die Plasmide pT7TS (GenBank-Zugriffsnummer U26404; Lai et al., Development 1995, 121: 2349 bis 2360), die pGEM^{®}-Reihe, bspw. pGEM^{®}-1 (GenBank-Zugriffsnummer X65300; von Promega) und pSP64 (GenBank-Zugriffsnummer X65327) genannt werden (vgl. auch Mezei und Storts, Purification of PCR Products, in: Griffin und Griffin (Hrsg.), PCR Technology: Current Innovation, CRC Press, Boca Raton, FL, 2001).

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restriktionsendonukleasen ausgeschnitten.

Die modifizierte mRNA, welche zur Transfektion der Blutzellen verwendet werden kann, kann darüber hinaus eine 5'-Cap-Struktur (ein modifiziertes Guanosin-Nucleotid) aufweisen. Als Beispiele von Cap-Strukturen können m7G(5')ppp, 5'(A,G(5')ppp(5')A und G(5')ppp(5')G genannt werden.

Gemäß einer weiteren bevorzugten Ausführungsform der vorliegenden Erfindung enthält die modifizierte mRNA einen Poly(A⁺)-Schwanz von mindestens etwa 25, insbesondere mindestens etwa 30, vorzugsweise mindestens etwa 50 Nucleotiden, mehr bevorzugt mindestens etwa 70 Nucleotiden, besonders bevorzugt mindestens etwa 100 Nucleotiden. Der Poly(A⁺)-Schwanz kann jedoch auch 200 und mehr Nucleotide umfassen.

Für eine effiziente Translation der mRNA ist weiterhin eine wirksame Bindung der Ribosomen an die Ribosomen-Bindungsstelle (Kozak-Sequenz: GCCGCCAC-CAUGG, das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht.

Des weiteren ist es möglich, in die mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere (z.B. zwei) Peptide bzw. Polypeptide codiert, die unabhängig voneinander durch die Ribosomen in den PBMCs translatiert werden sollen ("multicistronische" oder "polycistronische" (bspw. bicistronische) mRNA). Beispiele erfindungsgemäß verwendbarer IRES-Sequenzen sind diejenigen aus Picomaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorliegenden Erfindung weist die mRNA in den 5'- und/oder 3'-nicht-translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des α- und β-Globingens, bspw. von *Homo sapiens* oder *Xenopus laevis,* genannt werden. Ein anderes Beispiel einer Stabilisierungssequenz weist die allgemeine Formel (C/U)CCANₓCCC(U/A)PyₓUC(C/U)CC auf, die im 3'UTR der sehr stabilen mRNA enthalten ist, die für α-Globin, α-(I)-Collagen, 15-Lipoxygenase oder für Tyrosin-Hydroxylase codiert (vgl. Holcik et al., Proc. Natl. Acad. Sci. USA 1997, 94: 2410 bis 2414). Selbstverständlich können derartige Stabilisierungssequenzen einzeln oder in Kombination miteinander als auch in Kombination mit anderen, einem Fachmann bekannten Stabilisierungssequenzen verwendet werden.

Zur weiteren Stabilisierung der mRNA ist es außerdem bevorzugt, daß diese mindestens ein Analoges natürlich vorkommender Nucleotide aufweist. Dies beruht auf der Tatsache, daß die in den Blutzellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseffizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt. Erfindungsgemäß können derartige Analoga in nicht-translatierten und translatierten Bereichen der modifizierten mRNA vorkommen.

Des weiteren kann der wirksame Transfer der, vorzugsweise modifizierten, mRNA in die Zellen dadurch verbessert werden, daß die mRNA vor der Transfektion der zuvor gewonnenen Blutzellen mit einem kationischen oder polykationischen Agens, insbesondere einem entsprechenden Peptid oder Protein, assoziiert oder daran gebunden ist. Daher liegt die mRNA vor der Transfektion der PBMCs bevorzugt mit einem derartigen Agens komplexiert oder kondensiert vor. Insbesondere ist dabei die Verwendung von Protamin als polykationischem, Nucleinsäurebindenden Protein besonders wirksam. Des weiteren ist die Verwendung anderer kationischer Peptide oder Proteine, wie Poly-L-Lysin, Poly-L-Arginin oder Histonen, ebenfalls möglich. Diese Vorgehensweise zur Stabilisierung der modifizierten mRNA ist in EP-A-1083232 beschrieben, deren diesbezüglicher Offenbarungsgehalt in die vorliegende Erfindung vollumfänglich eingeschlossen ist. Des Weiteren kann die zur mRNA zur Transfektion in die Zellen mit anderen Substanzen zum effizienten Transfer assoziiert sein bzw. damit vermischt sein. Als Beispiele hierfür sind der Einschluss in Mikro- oder Nanopartikel, insbesondere solche auf Basis von PLGA (Poly(D,L-Lactid-co-Glykolid)), und Lipide zu nennen.

Darüber hinaus kann erfindungsgemäß die mRNA neben dem antigenen oder dem gentherapeutisch wirksamen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-1, IL-2, IL-3, IL-4, IL-5, IL-6, IL-7, IL-8, IL-9, IL-10, IL-12, IFN-α, IFN-γ, GM-CFS und LT-α) oder Wachstumsfaktoren, wie hGH, codiert. Alternativ oder zusätzlich kann die mRNA, die zur Transfektion der Blutzellen oder hämatopoietischen Zellen, insbesondere roten Blutzellen, PBMCs, Granulocyten und/oder Blutplätchen, vorgesehen ist, auch für mindestens ein co-stimulierendes Molekül (z.B. CD40, CD80, CD86 oder 4-1BB-Ligand) und/oder mindestens einen Transkriptionsfaktor (z.B. NF-kappaB oder ICSBP (engl. "interferon consensus binding protein")), der für eine besonders effiziente Expression immunstimulierender Moleküle in den transfizierten Zellen sorgt, und/oder für mindestens einen Homing-Rezeptor (bspw. CCR7), der die transfizierten Zellen bspw. in die Lymphknoten leitet, und/oder mindestens ein Suizid-Molekül (z.B Herpes Simplex Virus Thymidinkinase (HSV-tk, Cytochrom P450 4B1 (cyp4B1) und/oder Folylpolyglutamatsynthase (FPGS)) codieren, das in den transfizierten Zellen exprimiert wird und ein ansonsten nicht aktives Prodrug in seine aktive Form überführt (z.B. Nucleosidanaloga wie Ganciclovir oder Acyclovir durch HSV-tk und/oder 4-Ipomeanol oder 2-Aminoanthracen durch cyp4B1) oder die Wirkung eines an sich schon wirksamen Chemotherapeutikums verstärkt (bspw. Alkylantien wie Methotrexat durch FPGS), und so einen nekrotischen und/oder apoptotischen Zelltod induziert, was zur Freisetzung des Zellinhalts führt, der das von der mRNA codierte Antigen einschließt.

Ein weiterer Gegenstand der vorliegenden Anmeldung betrifft ein Verfahren zur Herstellung der vorstehend definierten pharmazeutischen Zusammensetzung, umfassend die Schritte:
(a) Gewinnen von Blutzellen, und
(b) Transfizieren der Blutzellen *in vitro* mit mindestens einer mRNA, die mindestens einen für mindestens ein Antigen codierenden Bereich umfaßt.

Die Gewinnung von Blutzellen aus einem tierischen oder menschlichen Patienten erfolgt dabei bspw. nach Standardverfahren. So kann in einfacher Weise Vollblut durch Punktion eines geeigneten Gefäßes gewonnen werden. Serum erhält man in bekannter Weise durch Koagulation der festen Blutbestandteile. Als Beispiel einer angereicherten Teilpopulation von Blutzellen können PBMCs genannt werden. Diese werden üblicherweise nach einem Verfahren isoliert, das von Bøyum (Nature 204: 793-794, 1964; Scan. J. Lab. Clin. Invest. Suppl. 97, 1967) erstmals beschrieben wurde. Im allgemeinen wird dazu dem Individuum Blut entnommen, welches zur Dichtegradientenzentrifugation z.B. auf eine Lösung mit einer Dichte von 1,077 g/ml (25°C), enthaltend üblicherweise Ficoll und Natriumdiatrizoat, gegeben wird. Während der vorsichtigen Zentrifugation bei Raumtemperatur sammeln sich die PBMCs an der Ficoll/Blut-Grenzschicht an, während die roten Blutzellen und die übrigen weißen Blutzellen sedimentiert werden. Die Grenzschicht mit den PBMCs wird gewonnen und üblicherweise mit einem geeigneten Puffer, z.B. sterilem PBS, gewaschen. Vorzugsweise werden die PBMCs einer kurzen isotonischen Behandlung mit einer wässerigen Lösung von bspw. Ammoniumchlorid unterworfen. Schließlich werden die PBMCs noch ein oder mehrmals mit einem Puffer, wie PBS (steril), gewaschen. Die so erhaltenen Zellen können dann gegebenenfalls unter geeigneten Bedingungen, üblicherweise bei -70°C, bis zur weiteren Verwendung gelagert werden.

Erfindungsgemäß bevorzugt handelt es sich bei den Blutzellen unmittelbar vor der Transfektion um frische Blutzellen, d.h. es vergeht zwischen Gewinnung der Blutzellen (insbesondere einer Blutentnahme) im Schritt (a) und der Transfektion gemäß Schritt (b) nur eine kurze Zeit, bspw. weniger als 12 h, vorzugsweise weniger als 6 h, mehr bevorzugt weniger als 2 h, besonders bevorzugt weniger als 1 h. Die Transfektion der Blutzellen erfolgt ebenfalls nach gängigen Verfahren, bspw. mittels Elektroporation oder chemischen Verfahren, insbesondere Lipofektion.

Hinsichtlich bevorzugter Ausführungsformen der Blutzellen wird auf die vorstehenden Ausführungen in Bezug auf die pharmazeutische Zusammensetzung der vorliegenden Erfindung verwiesen. Ebenso wird hinsichtlich bevorzugter Ausführungsformen der mRNA im Schritt (b) des erfindungsgemäßen Herstellungsverfahrens auf die entsprechenden Ausführungsformen zur erfindungsgemäßen pharmazeutischen Zusammensetzung verwiesen.

Gemäß einer weiteren bevorzugten Ausführungsform werden die transfizierten Zellen nach Schritt (b) einem Schritt (c) zur Stimulation mit immunstimulierenden Agenzien, wie LPS, TNFα, Toll-like-Rezeptor-Liganden wie doppelsträngige RNA, stabilisierte oder CpG-DNA usw. unterworfen.

Als ein Beispiel erfindungsgemäß verwendbarer Blutzellen können PBMCs, wie bereits vorstehend ausführlich dargelegt, durch Ficoll-Hypaque-Dichtegradientenzentrifugation gewonnen werden, wobei, falls erforderlich, ein oder mehrere nachfolgenden Waschschritte mit Phosphat-gepufferter Salzlösung (PBS) durchgeführt werden.

Die Herstellung der mRNA zur erfindungsgemäßen Transfektion erfolgt durch einem Fachmann bekannte Verfahren, insbesondere durch chemische Synthese oder mehr bevorzugt mittels molekularbiologischer Verfahren, die bereits vorstehend erwähnt sind.

Wie bereits vorstehend erläutert, liegt die mRNA bei der *In vitro*-Transfektion gemäß Schritt (b) mit mindestens einem kationischen oder polykationischen Agens komplexiert oder konvensiert vor, um die Stabilität der mRNA zu erhöhen, was zu einer besseren Transfektionseffizienz und insbesondere erhöhten Expressionsrate in den transfizierten Zellen führt. Geeignete kationische oder polykationische Agenzien sind bspw. Protamin, Poly-L-Lysin, Poly-L-Arginin oder Histone (vgl. hierzu den Offenbarungsgehalt der bereits oben zitierten EP-A-1083232).

Gemäß einer bevorzugten Ausführungsform umfaßt das obige Verfahren zur Herstellung der vorstehend definierten pharmazeutischen Zusammensetzung die Schritte:
(1) Herstellen einer cDNA-Bibliothek oder eines Teils davon aus Tumorgewerbe oder aus mit einem pathogenen Agens infizierten Zellen eines Patienten,
(2) Herstellen einer Matrize für die *In vitro* -Transkription von RNA anhand der cDNA-Bibliothek oder eines Teils davon und
(3) *In vitro* -Transkribieren der Matrize.

Das Tumorgewebe des Patienten kann bspw. durch eine einfache Biopsie gewonnen werden. Es kann aber auch durch operative Entfernung von Tumorbefallenem Gewebe bereitgestellt werden. Desgleichen können mit einem pathogenen Agens (erfindungsgemäß können Zellen selbstverständlich auch mit mehreren Keimen befallen sein) infizierte Zellen aus Biopsien irgendeines infizierten Gewebes, z.B. den Lmyphknoten, gewonnen werden. Als weitere besonders geeignete Quellen von infizierten Zellen können bspw. Blut, Serum, Lymphflüssigkeit und Synovialflüssigkeit genannt werden. Des Weiteren können infizierte Zellen gegebenenfalls auch aus anderen Körperflüssigkeiten, wie Sputum, Sperma, Vaginalflüssigkeit, Urin, Stuhl, Schweiß usw. gewonnen werden. Des Weiteren kann die Herstellung der cDNA-Bibliothek oder eines Teils davon gemäß Schritt (1) der bevorzugten Ausführungsform des Herstellungsverfahrens der vorliegenden Erfindung durchgeführt werden, nachdem das entsprechende Gewebe bzw. die Zellen zur Lagerung, vorzugsweise auf Temperaturen unterhalb von -70 °C, tiefgefroren wurde.

Zur Herstellung der cDNA-Bibliothek oder eines Teils davon wird zunächst eine Isolierung der Gesamt-RNA aus den Tumorgewebszelln bspw. aus den infizierten Zellen durchgeführt. Verfahren hierzu sind bspw. in Maniatis et al., *sup*ra, beschrieben. Des Weiteren sind hierfür entsprechende Kits im Handel, bspw. bei der Fa. Roche AG (z.B. das Produkt "High Pure RNA Isolation Kit"), erhältlich. Aus der Gesamt-RNA wird gemäß einem Fachmann bekannter Verfahren (vgl. bspw. Maniatis et al., *supra*) die entsprechende Poly(A⁺)-RNA isoliert. Auch hierfür sind im Handel entsprechende Kits erhältlich. Ein Beispiel ist das "High Pure RNA Tissue Kit" der Fa. Roche AG. Ausgehend von der so gewonnenen Poly(A⁺)-RNA wird danach die cDNA-Bibliothek hergestellt (vgl. auch hierzu bspw. Maniatis et al., *supra*). Auch für diesen Schritt bei der Herstellung der cDNA-Bibliothek stehen einem Fachmann im Handel erhältliche Kits, bspw. das "SMART PCR cDNA Synthesis Kit" der Fa. Clontech Inc., zur Verfügung.

Gemäß Schritt (2) der vorstehend dargestellten bevorzugten Ausführungsform des Herstellungsverfahrens wird ausgehend von der cDNA-Bibliothek (oder eines Teils davon) eine Matrize für die *In vitro* -Transkription synthetisiert. Dies erfolgt erfindungsgemäß insbesondere dadurch, daß die erhaltenen cDNA-Fragmente in einen geeigneten RNA-Produktionsvektor cloniert werden. Die geeignete DNA-Matrize und erfindungsgemäß bevorzugte Plasmide sind bereits vorstehend im Zusammenhang mit der Herstellung der zur Transfektion der Zellen vorgesehenen mRNA angegeben.

Zur *In vitro* -Transkription der im obigen Schritt (2) hergestellten Matrize wird diese, wenn sie als zirkuläre Plasmid-(c)DNA vorliegt, zunächst mit einem entsprechenden Restriktionsenzym linearisiert. Vorzugsweise wird das so geschnittene Konstrukt vor der eigentlichen *In vitro* -Transkription noch einmal gereinigt, bspw. durch entsprechende Phenol-Chloroform- und/oder Chloroform/Phenol/lsoamylalkohol-Gemische. Hierdurch wird insbesondere sichergestellt, daß die DNA-Matrize in proteinfreier Form vorliegt. Als nächstes erfolgt die enzymatische Synthese der RNA ausgehend von der gereinigten Matrize. Dieser Unterschritt erfolgt in einem entsprechenden Reaktionsgemisch, enthaltend die linearisierte, proteinfreie DNA-Matrize in einem geeigneten Puffer, dem vorzugsweise ein Ribonuclease-Inhibitor zugesetzt wird, unter Verwendung eines Gemischs der benötigten Ribonucleotidtriphosphate (rATP, rCTP, rUTP und rGTP) und einer ausreichenden Menge einer RNA-Polymerase, bspw. T7-Polymerase. Das Reaktionsgemisch liegt dabei in RNase-freiem Wasser vor. Bevorzugt wird bei der eigentlichen enzymatischen Synthese der RNA auch ein CAP-Analogon zugefügt. Nach einer entsprechend langen, bspw. 2 h, Inkubation bei 37°C, wird die DNA-Matrize durch Zugabe von RNase-freier DNase abgebaut, wobei bevorzugt wieder bei 37°C inkubiert wird.

Vorzugsweise wird die so hergestellte RNA mittels Ammoniumacetat/Ethanol gefällt und gegebenenfalls ein oder mehrmals mit RNase-freiem Ethanol gewaschen. Schließlich wird die so gereinigte RNA getrocknet und gemäß einer bevorzugten Ausführungsform in RNase-freiem Wasser aufgenommen. Des Weiteren kann die so hergestellte RNA mehreren Extraktionen mit Phenol/Chloroform bzw. Phenol/Chloroform/Isoamylalkohol unterworfen werden.

Gemäß einer weiteren bevorzugten Ausführungsform der vorliegenden Erfindung wird nur ein Teil einer Gesamt-cDNA-Bibliothek gewonnen und in entsprechende mRNA-Moleküle überführt. Daher kann erfindungsgemäß auch eine sog. Subtraktionsbibliothek als Teil der Gesamt-cDNA-Bibliothek verwendet werden, um die erfindungsgemäßen mRNA-Moleküle bereitzustellen. Ein bevorzugter Teil der cDNA-Bibliothek des Tumorgewebes codiert für die tumorspezifischen Antigene. Bei bestimmten Tumoren sind die entsprechenden Antigene bekannt. Bei mit einem pathogenen Agens (oder mehreren verschiedenen davon) infizierten Zellen codiert die cDNA-Teilbibliothek bevorzugt für die Produkte (Polypeptide) der aufgrund der Infektion hochregulierten Gene der (Wirts-)Zelle. Es kann gemäß einer weiteren bevorzugten Ausführungsform der für die tumorspezifischen Antigene codierende Teil der cDNA-Bibliothek bzw. der für die hochregulierten Genprodukte codierende Teil der cDNA-Bibliothek zunächst ermittelt werden (bspw. vor Schritt (1) des vorstehend definierten Verfahrens). Dies erfolgt vorzugsweise dadurch, daß die Sequenzen der tumorspezifischen Antigene bzw. der aufgrund der Infektion mit dem pathogenen Agens hochregulierten Genprodukte durch einen Abgleich mit einer entsprechenden cDNA-Bibliothek aus gesundem Gewebe bzw. nicht infizierten Zellen festgestellt werden.

Der erfindungsgemäße Abgleich umfasst insbesondere einen Vergleich der Expressionsmuster des gesunden Gewebes mit dem des in Rede stehenden Tumorgewebes bzw. im Fall der Immunstimulation gegen einen pathogenen Keim einen Vergleich der Expressionsmuster gesunder Zellen mit infizierten Zellen. Entsprechende Expressionsmuster können auf Nukleinsäureebene bspw. mit Hilfe geeigneter Hybridisierungsexperimnte bestimmt werden. Hierzu können bspw. die entsprechenden (m)RNA- oder cDNA-Bibliotheken der Gewebe bzw. Zellen jeweils in geeigneten Agarose- oder Polyacrylamid-Genen aufgetrennt, auf Membranen überführt und mit entsprechenden Nukleinsäure-Sonden, vorzugsweise Oligonukleotid-Sonden, welche die jeweiligen Gene repräsentieren, hybridisiert werden (Northem- bzw. Southem-Blots). Ein Vergleich der entsprechenden Hybridisierungen liefert somit die zur Immunstimulation besonders geeigneten Gene, also ausschließlich vom Tumorgewebe oder darin stärker exprimierte Gene bzw. in infizierten Zellen hochregulierte Gene.

Gemäß einer weiteren bevorzugten Ausführungsform werden die genannten Hybridisierungsexperimente mit Hilfe einer Diagnose durch Mikroarrays (ein oder mehrere Mikroarrays) durchgeführt. Ein entsprechender DNA-Mikroarray umfaßt eine definierte Anordnung, insbesondere auf kleinem oder kleinstem Raum, von Nukleinsäure-, insbesondere Oligonukleotid-Sonden, wobei jede Sonde bspw. jeweils ein Gen, dessen Anwesenheit oder Abwesenheit in der entsprechenden (m)RNA- oder cDNA-Bibliothek zu untersuchen ist, repräsentiert. In einer entsprechenden Mikroanordnung können so Hunderte, Tausende und sogar Zehn- bis Hunderttausende von Genen repräsentiert sein. Zur Analyse des Expressionsmusters des jeweiligen Gewebes bzw. der jeweiligen Zellen wird dann entweder die Poly(A⁺)-RNA oder, was bevorzugt ist, die entsprechende cDNA mit einem geeigneten Marker, insbesondere werden hierzu Fluoreszenzmarker verwendet, markiert und unter geeigneten Hybridisierungsbedingungen mit dem Mikroarray in Kontakt gebracht. Bindet eine cDNA-Spezies an ein auf dem Mikroarray vorhandenen Sondenmolekül, insbesondere einem Oligonukleotid-Sondenmolekül, wird dementsprechend ein mehr oder minder stark ausgeprägtes Fluoreszenzsignal, das mit einem geeigneten Detektionsgerät, bspw. einem entsprechend ausgelegten Fluoreszenzspektrometer, gemessen werden kann, beobachtet. Je häufiger die cDNA (oder RNA) -Spezies in der Bibliothek repräsentiert ist, desto stärker wird das Signal, bspw. das Fluoreszenzsignal, sein. Das entsprechende Mikroarray-Hybridisierungsexperiment (bzw. mehrere oder viele davon) wird (werden) getrennt für das Tumorgewebe und das gesunde Gewebe bzw. für die infizierten und nicht-infizierten Zellen durchgeführt. Die Differenz der aus den Mikroarray-Experimenten ausgelesenen Signale läßt daher auf die ausschließlich oder vermehrt vom Tumorgewebe exprimierten Gene bzw. auf die aufgrund der Infektion mit dem pathogenen Agens hochregulierten Gene schließen. Derartige DNA-Mikroarray-Analysen sind bspw. in Schena (2003), Mikroarray Analysis, ISBN 0-471-41443-3, John Wiley & Sons, Inc., New York, dargestellt, wobei der diesbezügliche Offenbarungsgehalt dieser Druckschrift vollumfänglich in die vorliegende Erfindung aufgenommen ist.

Die Erstellung infektions- bzw. tumorgewebsspezifischer Expressionsmuster ist jedoch keineswegs auf Analysen auf Nukleinsäureebene beschränkt. Einem Fachmann sind selbstverständlich auch im Stand der Technik bekannte Verfahren geläufig, welche der Expressionsanalyse auf Proteinebene dienen. Hier sind insbesondere Techniken der 2D-Gelelektrophorese und der Massensprektrometrie zu nennen, wobei diese Techniken vorteilhafterweise auch mit Proteinbiochips (also Mikroarrays auf Proteinebene, bei denen bspw. ein Proteinextrakt aus gesundem bzw. Tumorgewebe oder aus einer infizierten bzw. einer nicht-infizierten Zellen mit auf dem Mikroarray-Substrat aufgetragenen Antikörpern und/oder Peptiden in Kontakt gebracht wird), kombiniert werden können. Hinsichtlich der massenspektroskopischen Verfahren sind diesbezüglich insbesondere MALDI-TOF-("matrix assisted laser desorption/ionisation-time of flight"-) Verfahren zu nennen. Die genannten Techniken zur proteinchemischen Analyse zur Gewinnung des Expressionsmusters von Tumor- im Vergleich zu gesundem Gewebe bzw. infizierten Zellen im Vergleich zu entsprechenden nicht-infizierten Zellen sind bspw. in Rehm (2000) Der Experimentator: Proteinbiochemie/Proteomics, Spektrum Akademischer Verlag, Heidelberg, 3. Aufl., beschrieben, auf dessen diesebezüglichen Offenbarungsgehalt in der vorliegenden Erfindung *expressis verbis* Bezug genommen wird. Hinsichtlich Protein-Mikroarrays wird außerdem wiederum auf die diesbezüglichen Ausführungen in Schena (2003), *supra,* verwiesen.

Die erfindungsgemäße pharmazeutische Zusammensetzung enthält neben den transfizierten Zellen einen oder mehrere pharmazeutisch verträgliche(n) Träger und/oder ein oder mehrere pharmazeutisch verträgliche(s) Vehikel. Geeignete Träger bzw. Vehikel sind vorzugsweise an die jeweiligen Blutzellen angepasste sterile Medien oder Pufferlösungen.

Wege zur geeigneten Formulierung und Herstellung der erfindungsgemäßen pharmazeutischen Zusammensetzung sind in "Remington's Pharmaceutical Sciences" (Mack Pub. Co., Easton, PA, 1980) offenbart, das vollinhaltlich Bestandteil der Offenbarung der vorliegenden Erfindung ist. Für die parenterale Verabreichung kommen als Trägerstoffe bspw. neben sterilem Wasser, sterilen Pufferlösungen oder sterilen Medien auch Polyalkylenglykole, hydrierte Naphthalene und insbesondere biokompatible Lactidpolymere, Lactid/Glycolidcopolymer oder Polyoxyethylen-/Polyoxypropylencopolymere in Betracht. Erfindungsgemäße Zusammensetzungen können Füllsubstanzen oder Substanzen, wie Lactose, Mannitol, Substanzen zur kovalenten Anknüpfung von Polymeren, wie z.B. Polyethylenglykol, Komplexierung mit Metallionen oder Einschluß von Materialien in oder auf besonderen Präparationen von Polymerverbindungen, wie z.B. Polylactat, Polyglykolsäure, Hydrogel oder auf Liposomen, Mikroemulsionen, Micellen, unilamellare oder multilamellare Vesikel, Erythrocyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wirkstoffkomponenten in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Polyoxamere oder Polyoxamine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte wässerige Trägermaterialien sind z.B. Wasser zur Injektion (WFI) oder Wasser, gepuffert mit Phosphat, Citrat oder Acetat usw., bzw. entsprechende Zellmedien, wobei der pH typischerweise auf 5,0 bis 8,0, vorzugsweise 6,0 bis 7,0, eingestellt wird. Der oder die Träger bzw. das oder die Vehikel wird/werden zusätzlich vorzugsweise Salzbestandteile enthalten, z.B. Natriumchlorid, Kaliumchlorid oder andere Komponenten, welche die Lösung bspw. isotonisch machen. Weiterhin kann/können der oder die Träger bzw. das oder die Vehikel neben den vorstehend genannten Bestandteilen zusätzliche Komponenten, wie fötales Kälberserum, Wachstumsfaktoren, humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortschrittsstadium, wie auch dem Körpergewicht, dem Alter und dem Geschlecht des Patienten ab.

Die Konzentration der transfizierten Zellen in derartigen Formulierungen kann daher innerhalb eines weiten Bereichs von bspw. 5 x 10⁴ bis 1 x 10⁸ Zellen/ml variieren. Die erfindungsgemäße pharmazeutische Zusammensetzung wird vorzugsweise parenteral, bspw. intravenös, intraarteriell, subkutan, intramuskulär, dem Patienten verabreicht. Des Weiteren kann die pharmazeutische Zusammensetzung der vorliegenden Erfindung lokal, bspw. in einen Tumor, injiziert werden.

Somit wird erfindungsgemäß auch ein Verfahren zur Behandlung bzw. ein Impfverfahren zur Prävention von Krebserkrankungen oder Infektionserkrankungen, bspw. der vorstehend genannten Erkrankungen, bereitgestellt, welches das Verabreichen der erfindungsgemäßen pharmazeutischen Zusammensetzung an einen Patienten, insbesondere einen Menschen, umfasst.

Gemäß einer bevorzugten Ausführungsform des Behandlungs- bzw. Impfverfahrens bzw. bei der vorstehend definierten Verwendung der erfindungsgemäß transfizierten Blutzellen, d.h. eines Gemsiches von roten Blutzellen, Granulocyten, mononucleären Zellen und/oder Blutplättchen bzw. einer angereicherten oder im wesentlichen reinen Teilpopulation davon, zur Herstellung einer pharmazeutischen Zusammensetzung zur Behandlung und/oder Prävention von Krebserkrankungen oder Infektionserkrankungen wird/werden dem Patienten neben der erfindungsgemäßen pharmazeutischen Zusammensetzung ein oder mehrere Cytokin(e) und/oder ein oder mehrere co-stimulierende Molekül(e) verabreicht. Hinsichtlich besonders geeigneter zusätzlich verabreichter Spezies vgl. die obigen Ausführungen hinsichtlich der neben dem oder den Antigen(en) von der mRNA zusätzlich codierten Moleküle.

Daher wird erfindungsgemäß auch allgemein ein Behandlungs- bzw. Impfverfahren bereitgestellt, umfassend das Verabreichen der erfindungsgemäß transfizierten Blutzellen und mindestens eines Cytokins, bspw. eines oder mehrerer der vorstehend genannten Cytokine, insbesondere GM-CSF, an einen Patienten, insbesondere einen Menschen. Das Verfahren dient insbesondere zur Behandlung und/oder Prävention entsprechender Krebserkrankungen (bspw. die obigen Krebserkrankungen) oder Infektionserkrankungen. Dementsprechend ist die vorliegende Erfindung auch allgemein auf eine pharmazeutische Zusammensetzung gerichtet, umfassend transfizierte Blutzellen (nach vorstehender Definition) und mindestens ein Cytokin, bspw. eines oder mehrerer der vorstehend genannten Cytokine, wie GM-CSF, vorzugsweise in Verbindung mit einem pharmazeutisch verträglichen Träger und/oder Vehikel. Erfindungsgemäß wird somit auch die Verwendung von Cytokinen, bspw. eines oder mehrerer der vorstehend genannten Cytokine, insbesondere GM-CSF, in Kombination mit den transfizierten Blutzellen obiger Definition zur Behandlung und/oder Prävention von Krebserkrankungen (z.B. vorstehend angeführter Krebserkrankungen) oder Infektionserkrankungen (bspw. den vorstehend aufgeführten Infektionserkrankungen) offenbart.

Gemäß einer weiteren bevorzugten Ausführungsform der vorliegenden Erfindung wird das Cytokin, bspw. GM-CSF, gleichzeitig oder, was mehr bevorzugt ist, vor oder nach der pharmazeutischen Zusammensetzung, enthaltend die erfindungsgemäß transfizierten Zellen, verabreicht (bzw. zur Herstellung eines entsprechendes Arzneimittels zur gleichzeitigen Verabreichung mit oder zur Verabreichung vor oder nach den vorstehend aufgeführten Blutzellen verwendet). Ganz besonders bevorzugt erfolgt die Verabreichung des Cytokins, insbesondere GM-CSF, kurz vor (z.B. etwa 2 h oder weniger, bspw. bis etwa 5 min) oder kürzere Zeit (bspw. etwa 5, 10, 15, 30, 45 oder 60 min) nach oder längere Zeit (z.B. etwa 2, 6, 12, 24 oder 36 h) nach der Verabreichung der vorstehend definierten pharmazeutischen Zusammensetzung bzw. allgemein nach den erfindungsgemäß transfizierten Zellen.

Die Applikation des Cytokins, bspw. GM-CFS, kann dabei auf dem gleichen Wege wie die erfindungsgemäßen pharmazeutischen Zusammensetzungen bzw. die erfindungsgemäß transfizierten Blutzellen oder in einer davon getrennten Weise erfolgen. Geeignete Verabreichungswege sowie auch die geeigneten Formulierungsmöglichkeiten in Bezug auf das oder die Cytokin(e) können den obigen Ausführungen hinsichtlich der erfindungsgemäßen pharmazeutischen Zusammensetzungen entnommen werden. Bei einem humanen Patienten ist insbesondere eine GM-CFS-Dosis von 100 Mikrogramm/m² empfehlenswert. Besonders bevorzugt erfolgt die Verabreichung des Cytokins, bspw. GM-CFS, durch eine s.c.-Injektion.

Vorzugsweise werden die pharmazeutischen Zusammensetzungen der vorliegenden Erfindung bzw. die erfindungsgemäß transfizierten Blutzellen und gegebenenfalls damit zusammenhängend das oder die Cytokin(e) bzw. andere co-stimulierende Moleküle in Form von Intervall-Dosen appliziert. Beispielsweise kann eine Dosis einer erfindungsgemäßen pharmazeutischen Zusammensetzung in kürzeren Intervallen, bspw. täglich, jeden zweiten Tag, jeden dritten Tag usw., oder aber, was mehr bevorzugt ist, in längeren Intervallen, bspw. einmal wöchentlich, einmal in zwei Wochen, einmal in drei Wochen, einmal im Monat usw. verabreicht werden. Dabei können die Intervalle auch veränderlich sein, wobei insbesondere die immunologischen Parameter des Patienten zu berücksichtigen sind. Bspw. kann die Verabreichung einer erfindungsgemäßen pharmazeutischen Zusammensetzung (und gegebenenfalls damit zusammenhängend auch die Verabreichung des oder der Cytokins/Cytokine bzw. des oder der co-stimulierenden Moleküls/Moleküle) einem Behandlungsschema folgen, bei dem zu Beginn der Behandlung das Intervall kürzer ist, bspw. einmal in zwei Wochen, und dann, je nach Behandlungsverlauf bzw. den entsprechend bestimmten immunologischen Parametern des Patienten, das Intervall auf bspw. einmal im Monat verlängert wird. Je nach Patient, insbesondere dessen Zustand und seinen immunologischen Parametern, kann so ein auf das jeweilige Individuum zugeschnittenes Therapieschema angewandt werden.

Erfindungsgemäß wird insgesamt daher auch ein Behandlungsverfahren bereitgestellt, bei dem zunächst Blutzellen bzw. hämatopoietische Zellen (gemäß vorstehender Definition) aus einem Patienten (Tier oder Mensch) gewonnen, erfindungsgemäß mit der obig definierten mRNA *in vitro* transfiziert und schließlich einem entsprechenden Patienten, vorzugsweise demselben Patienten, welchem die jeweiligen Blutzellen im ersten Schritt entnommen wurden, verabreicht werden, wobei auf die diesbezüglichen Ausführungen hinsichtlich der Formulierung und Verabreichung der erfindungsgemäßen pharmazeutischen Zusammensetzung Bezug genommen wird.

Erfindungsgemäß werden also Blutzellen bzw. hämatopoietische Zellen, die mit mindestens einer mRNA transfiziert sind, welche mindestens einen für mindestens ein Antigen codierenden Bereich umfasst, zur Stimulation einer Immunantwort gegen das oder die von der mRNA codierte(n) Antigen(e) verwendet (bzw. zur Herstellung eines entsprechenden Arzneimittels zur Immunstimulation verwendet).

Erfindungsgemäß wurde daher überraschend festgestellt, daß es zur Vakzinierung gegen bestimmte Antigene nicht erforderlich ist, geeignete Blutzellen vor der Transfektion mit einer für das jeweilige Antigen codierenden mRNA mittels aufwendiger Zellkulturtechniken in Antigen-präsentierende Zellen (APCs), insbesondere dendritische Zellen (DCs) zu differenzieren, um eine entsprechende Immunantwort im Patienten auszulösen. APCs zeichnen sich insbesondere dadurch aus, daß sie durch die Expression co-stimulierender Moleküle und die Sekretion von Cytokinen mit Lymphocyten wechselwirken und über diese eine Antigen-spezifische Immunantwort auslösen können. Neben DCs sind auch Makrophagen und B-Lymphocyten APCs. Erfindungsgemäß verwendete Blutzellen enthalten B-Zellen, Monozyten, T-Lymphozyten, gegebenenfalls Granulocyten und eine geringe Anzahl DCs, die sich in plasmacytoide (pDC) und myeloide (mDC) DCs unterscheiden lassen. Ohne an eine bestimmte Theorie der Wirkungsweise der so transfizierten Blutzellen gebunden zu sein, wird erfindungsgemäß nach derzeitigem Wissensstand angenommen, daß die dem Patienten bspw. durch Injektion verabreichen transfizierten Zellen das durch die mRNA codierte Protein exprimieren und einerseits direkt (durch die in den Blutzellen wie den PBMCs vorhandenen APCs) oder indirekt (durch die transfizierten Zellen, die keine APCs sind, jedoch absterben und von im Organismus vorhandenen APCs durch Phagocytose aufgenommen werden oder durch von den transfizierten Zellen sekretierte Proteinen, die durch von im Organismus vorhandene APCs mittels Phagocytose aufgenommen werden) eine Antigen-spezifische Immunität stimulieren. Im Gegensatz zum Stand der Technik sind somit bei der vorliegenden Erfindung keine aufwendigen Verfahrensschritte, bspw. Zellkulturschritte u.ä, erforderlich, um angereicherte APC-Populationen zu gewinnen oder auf andere Weise z.B. artifizielle APCs herzustellen. Darüber hinaus müssen keine Cytokine in großen Mengen eingesetzt werden. Typischerweise vergehen erfindungsgemäß lediglich eine bis wenige Stunden, bspw. 2 Stunden, zwischen der Gewinnung der Blutzellen, z.B. der Blutentnahme, bis zur Verabreichung der bspw. als Tumor- oder Infektionsvakzine verwendeten pharmazeutischen Zusammensetzung. Es werden erfindungsgemäß im Gegensatz zu bisher bekannten Verfahren Blutzellen, i.a. ein Gemisch von roten Blutzellen, mononucleären Zellen, Granulocyten und/oder Blutplättchen bzw. angereicherte oder im wesentlichen reine Populationen dieser Blutzellen, und nicht *in vitro* generierte APCs verabreicht, was zu den vorstehend aufgeführten Vorteilen führt.

Die Figuren zeigen:

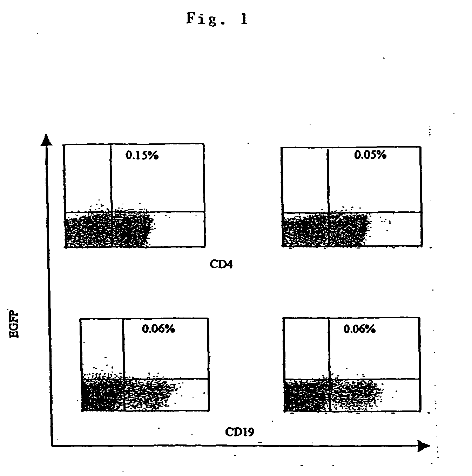

- Fig. 1: zeigt eine graphische Darstellung der Ergebnisse von FACS-Experimenten hinsichtlich der Expression von EGFP in CD4-spezifischen T-Helferzellen bzw. CD19-spezifischen B-Zellen. EGFP-codierende mRNA (linke Grafi- ken) oder für das Influenza-Matrixprotein codierende mRNA (Kontrolle; rechte Grafiken) wurde *in vitro* durch Elektroporation in frische humane PBMCs transfiziert. Die EGFP-Expression wurde danach aufgrund der Flu- oreszenz des EGFP mittels FACS untersucht. Die Detektion CD4- bzw. CD19-positiver Zellen erfolgte mittels fluoreszierender Anti-CD4- (obere Grafiken) bzw. Anti-CD19-Antikörper (untere Grafiken). Die Ergebnisse zeigen, daß einige der in den PBMCs enthaltenen CD4-positiven Zellen die mRNA aufgenommen haben und EGFP exprimieren.

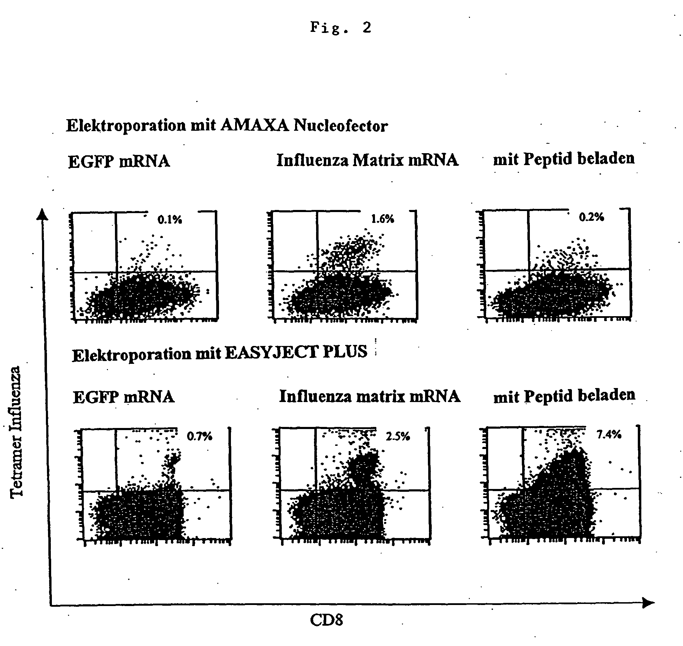

- Fig. 2: zeigt ebenfalls graphische Darstellungen der Ergebnisse von FACS- Experimenten, die belegen, daß mit mRNA transfizierte PBMCs T-Zellen gegen von der mRNA codierte Antigene aktivieren können. Die Experimen- te wurden für 2 verschiedene Elektroporationsverfahren, einerseits mit dem Nucleofector-Gerät der Fa. AMAXA und mit dem Standard- Elektroporationssystem EASYJECT PLUS der Fa. Equibio andererseits durchgeführt. Frische humane PBMCs wurden entweder mit EGFP-mRNA oder mit einer mRNA, die für das Influenza-Matrixprotein codiert, transfi- ziert. Als Positivkontrolle dienten mit dem Peptid GILGFVFPL des Influen- za-Matrixproteins beladene PBMCs. Nach der Transfektion bzw. im Fall der Positivkontolle nach der Beladung wurden die Zellen für eine Woche mit frischen autologen PBMCs co-kultiviert. Dann wurden die Zellen mit Hilfe PercP-markierter CD8-spezifischer monoklonaler Antikörper (zur Detektion von cytotoxischen T-Zellen) und durch PE-Markierung fluoreszierenden MHC-Tetrameren (welche ausschließlich diejenigen cytotoxischen T-Zellen erkennen, die für das Epitop des Influenza-Matrixproteins spezifisch sind, das durch HLA-A*0201 präsentiert wird) markiert. Bei beiden Elektroporati- onsverfahren wird festgestellt, daß nur 0,1 bzw. 0,7% der CD8-positiven cytotoxischen T-Zellen für das dominante Influenza-Matrixprotein-Epitop spezifisch sind, wenn die zur Stimulation verwendeten PBMCs mit EGFP- mRNA transfiziert wurden. Im Gegensatz dazu wird bei der Transfektion von PBMCs mit der mRNA, die für das Influenza-Matrixprotein codiert; nach einer Woche eine Frequenz von CD8-positiven cytotoxischen T- Zellen, die für das dominante Influenza-Matrixprotein-Epitop spezifisch sind, von 1,6 % bzw. 2,5 % beobachtet. Daher kann erfindungsgemäß festgestellt werden, daß die Elektroporation von PBMCs mit mRNA, die für das Influenza-Matrixprotein codiert, nicht jedoch durch die Transfektion mit EGFP-mRNA, die Poliferation von cytoxischen T-Zellen, die für das domi- nante Influenza-Matrixprotein-Epitop spezifisch sind, induziert wird. Dieses Ergebnis ist darüber hinaus unabhängig von der Art der Elektroporation.

Die vorliegende Erfindung wird durch die folgenden nicht-einschränkenden Beispiele näher erläutert.

### Beispiel 1

### Isolierung von PBMCs

Mononucleäre Zellen des peripheren Blutes wurden von gesunden HLA-A0201-positiven Spendern isoliert. Mononucleäre Zellen wurden durch Ficoll-Hypaque-Gradientenzentrifugation gewonnen. Die erhaltenen PBMCs wurden dreifach mit PBS gewaschen.

### Beispiel 2

### Transfektion von PBMCs durch Elektroporation

Die erhaltenen PBMCs wurden mit der Nucleofector-Vorrichtung und dem Human B-Cell Nucleofector Kit (beides AMAXA GmbH, Köln, Deutschland) gemäß den Angaben des Herstellers transfiziert. Pro Transfektion wurden 4 x 10⁶ Zellen mit jeweils 5 Mikrogramm RNA transfiziert.

Zur Immunophenotypisierung von PBMCs, die mit der EGFP-mRNA transfiziert wurden, wurden die monoklonalen Antikörper CD4-PerCP und CD19-PE verwendet (beide monoklonalen Antikörper von BD Pharmingen).

Nach der Transfektion wurden 1,5 x10⁶ Zellen in Kulturplatten mit 24 Vertiefungen (Greiner) in 1,5 ml X-Vivo 15 Medium (Bio Whittaker, Belgien), enthaltend 100 mg/ml LPS (Sigma, Deisenhofen, Deutschland) und 2,5 mg/ml TNF-α (R&D Systems), zur Reifung inkubiert. Als Positivkontrolle wurden nicht-transfizierte reife PBMCs mit 1 mg/ml des HLA-A*0201-restringiertem Peptids (GILGFVFTL) des Influenza-Matrixproteins für 1 h beladen. Nach der Inkubation über 24 h wurden die reifen PBMCs mit Medium gewaschen. Dann wurden die Zellen zur Stimulation von syngenen aufgetauten PBMCs (10⁷ Zellen pro Vertiefung) verwendet. Nach 4 Tagen wurden frisches Medium und 10 U/ml rekombinantes IL-2 und 5 mg/ml rekombinantes IL-7 (beide R&D Systems) zugegeben. Nach 6 Tagen Kultur wurden die Zellen mit einem PE-markierten humanen HLA-A*0201-Tetramer, das für das Influenza-Matrixprotein spezifisch ist, markiert. Zur Detektion von CD8-spezifischen Zellen wurde ein FITC-markierter Anti-CD4-Antikörper, gerichtet gegen einen PercP-markierten CD-8-Antikörper, verwendet.

### Beispiel 3

### Expression von EGFP in in vitro transfizierten humanen PBMCs

Für EGFP codierende mRNA wurde durch Elektroporation (oder durch Lipofektion, Daten nicht gezeigt) in frische humane PBMCs transfiziert. Einen Tag nach der Transfektion wurde die Expression von EGFP in Zellen, die mit fluoreszierenden monoklonalen Antikörpern (Anti-CD4 für T-Helferzellen oder Anti-CD19 für B-Zellen) durch FACS-Analyse untersucht. Wie in der Fig. 1 gezeigt, haben einige CD4-positive Zellen die mRNA aufgenommen und EGFP exprimiert. In einigen Fällen konnte auch eine EGFP-Expression in B-Zellen sowie auch in anderen Zellen, die keine B- bzw. T-Zellen sind, bspw. Monocyten, festgestellt werden (nicht gezeigt).

### Beispiel 4

### Mit mRNA transfizierte PBMCs aktivieren T-Zellen

PBMCs von HLA-A*0201-positiven gesunden Spendern wurden mit mRNA, die für das Influenza-Matrixprotein codiert, transfiziert. Die Transfektionen wurden unter Verwendung der Nucleofector-Vorrichtung von AMAXA oder eines Standard-Elektroporationssystems (EASYJECT PLUS) der Fa. Equibio durchgeführt. Die transfizierten PBMCs wurden eine Woche zusammen mit autologen frischen PBMCs *in vitro* co-kultiviert, gegebenenfalls wurde über Nacht eine Reifung durch Inkubation mit LPS und TNF-α durchgeführt Die gleichen Ergebnisse wurden jedoch auch mit nicht-stimulierten transfizierten PBMCs erhalten. Danach wurden die Zellen unter Verwendung monoklonaler Antikörper (Anti-CD8 für CD8-positive cytotoxische T-Zellen) und fluoreszierenden MHC-Tetrameren markiert, wobei letztere ausschließlich diejenigen cytotoxischen T-Zellen erkennen, die für das durch HLA-A*0201 präsentierte Epitop des Influenza-Matrixproteins spezifisch sind. Wie die Ergebnisse der Fig. 2 zeigen, induziert nur die Elektroporation von mRNA, die für das Influenza-Matrixprotein codiert, eine Proliferation von cytotoxischen T-Zellen, die für das dominante, vom Influenza-Matrixprotein abgeleitete Epitop spezifisch sind, während die Transfektion von EGFP-mRNA keine entsprechende Proliferation CD8-positiver cytotoxischer T-Zellen hervorruft. Dieses Ergebnis ist im übrigen unabhängig vom verwendeten Elektroporationssystem und des weiteren unabhängig davon, ob die transfizierten PBMCs vor der Co-Kultivierung über Nacht mit LPS und TNF-α behandelt wurden oder nicht.

Des weiteren kann durch *In*-*vivo*-Experimente in Mäusen gezeigt werden, daß per Elektroporation transfizierte frische Splenocyten als Blutzellen im Sinne der vorliegenden Erfingung eine Immunantwort auslösen können. Dies gilt insbesondere auch für bi-cistronische mRNA, die für das interessierende Antigen und gleichzeitig für ein Cytokin, einen co-stimulierenden Rezeptor, ein Homing-Molekül und/oder ein Suizid-Molekül (das eine Nekrose oder Apoptose auslöst) codiert.

## Patentansprüche

1. Pharmazeutische Zusammensetzung, enthaltend mit mindestens einer mRNA transfizierte Blutzellen in Verbindung mit einem pharmazeutisch verträglichen Träger und/oder Vehikel, wobei die mRNA mindestens einen für mindestens ein Antigen codierenden Bereich umfasst, wobei die Blutzellen im Wesentlichen reine oder angereicherte PBMCs sind und nicht mehr als 5% dendritische Zellen (DCs) enthalten.

2. Pharmazeutische Zusammensetzung nach Anspruch 1, wobei die Blutzellen nicht mehr als 2% dendritische Zellen (DCs) enthalten.

3. Pharmazeutische Zusammensetzung nach Anspruch 1 oder 2, enthaltend autologe Blutzellen.

4. Pharmazeutische Zusammensetzung nach einem der Ansprüche 1 bis 3, wobei die mRNA einen Bereich umfasst, der für mindestens ein Antigen aus einem Tumor oder eines pathogenen Agens codiert.

5. Pharmazeutische Zusammensetzung nach Anspruch 4, wobei das oder die Antigen(e) ein Polyepitop von Antigenen aus einem Tumor oder eines pathogenen Agens ist/sind.

6. Pharmazeutische Zusammensetzung nach Anspruch 5, wobei das Polyepitop durch Deletion, Addition und/oder Substitution eines oder mehrerer Aminosäurereste modifiziert ist.

7. Pharmazeutische Zusammensetzung nach einem der Ansprüche 4 bis 6, wobei die Blutzellen mit einer Mehrzahl von mRNA-Molekülen transfiziert sind, welche eine cDNA-Bibliothek oder einen Teil davon eines Tumorgewebes oder einer mit einem pathogenen Agens infizierten Zelle repräsentieren.

8. Pharmazeutische Zusammensetzung nach Anspruch 7, wobei der Teil der cDNA-Bibliothek für die tumorspezifischen Antigene oder für die aufgrund der Infektion mit dem pathogenen Agens hochregulierten Genprodukte codiert.

9. Pharmazeutische Zusammensetzung nach einem der Ansprüche 4 bis 8, wobei das oder die Tumorantigen(e) aus der Gruppe, bestehend aus 707-AP, AFP, ART-4, BA-GE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R1701, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pml/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1, ausgewählt ist/sind.

10. Pharmazeutische Zusammensetzung nach einem der Ansprüche 4 bis 8, wobei das pathogene Agens aus der Gruppe, bestehend aus Viren, Bakterien und Protozoen, ausgewählt ist.

11. Pharmazeutische Zusammensetzung nach Anspruch 10, wobei das virale, bakterielle oder protozoologische Antigen aus einem sekretierten Protein stammt.

12. Pharmazeutische Zusammensetzung nach einem der Ansprüche 1 bis 11, wobei der für das oder die Antigen(e) codierende Bereich und/oder der 5'- und/oder der 3'-nicht-translatierte Bereich der mRNA gegenüber der Wildtyp-mRNA derart verändert ist, dass er keine destabilisierenden Sequenzelemente aufweist.

13. Pharmazeutische Zusammensetzung nach einem der Ansprüche 1 bis 12, wobei die mRNA einen Sequenzbereich enthält, welcher der Erhöhung der Translationsrate dient.

14. Pharmazeutische Zusammensetzung nach einem der Ansprüche 1 bis 13, wobei die mRNA zusätzlich für mindestens ein Cytokin und/oder mindestens ein costimulierendes Molekül und/oder mindestens einen Transkriptionsfaktor und/oder mindestens einen Homing-Rezeptor und/oder mindestens ein Suizidgen codiert.

15. Pharmazeutische Zusammensetzung nach einem der Ansprüche 1 bis 14, wobei die mRNA eine 5'-Cap-Struktur und/oder einen Poly(A⁺)-Schwanz von mindestens etwa 25 Nucleotiden und/oder mindestens eine IRES und/oder mindestens eine 5'-Stabilisierungssequenz und/oder mindestens eine 3'-Stabilisierungssequenz aufweist.

16. Pharmazeutische Zusammensetzung nach Anspruch 15, wobei die 5'- und/oder die 3'-Stabilisierungssequenz(en) aus der Gruppe, bestehend aus nicht-translatierten Sequenzen (UTR) des α- oder β-Globingens und einer Stabilisierungssequenz der allgemeinen Formel (C/U)CCANₓCCC(U/A)PyₓUC(C/U)CC, ausgewählt ist/sind.

17. Pharmazeutische Zusammensetzung nach einem der Ansprüche 1 bis 16, wobei die mRNA mindestens ein Analoges natürlich vorkommender Nucleotide aufweist.

18. Pharmazeutische Zusammensetzung nach Anspruch 17, wobei das Analoge aus der Gruppe, bestehend aus Phosphorthioaten, Phosphoramidaten, Peptidnucleotiden, Methylphosphonaten, 7-Deazaguanosin, 5-Methylcytosin und Inosin, ausgewählt ist.

19. Pharmazeutische Zusammensetzung nach einem der Ansprüche 1 bis 18 zur Therapie und/oder Prophylaxe gegen Krebs oder Infektionskrankheiten.

20. Verfahren zur Herstellung einer pharmazeutischen Zusammensetzung nach einem der Ansprüche 1 bis 19, umfassend die folgenden Schritte:
(a) Gewinnen von Blutzellen, wobei die Blutzellen im Wesentlichen reine oder angereicherte PBMCs sind und nicht mehr als 5% dendritische Zellen (DCs) enthalten und
(b) Transfizieren der Blutzellen *in vitro* mit mindestens einer mRNA, die mindestens einen für mindestens ein Antigen codierenden Bereich umfasst.

21. Verfahren nach Anspruch 20, wobei die Blutzellen unmittelbar vor der Transfektion gemäß Schritt (b) nicht mehr als 2 % DCs enthalten.

22. Verfahren nach Anspruch 20 oder 21, wobei zwischen der Gewinnung der Blutzellen im Schritt (a) und der Transfektion im Schritt (b) weniger als 12 h, vorzugsweise weniger als 6 h, insbesondere bevorzugt weniger als 2 h vergehen.

23. Verfahren nach einem der Ansprüche 20 bis 22, wobei die Blutzellen autolog sind.

24. Verfahren nach einem der Ansprüche 20 bis 23, wobei die mRNA wie in einem der Ansprüche 4 bis 19 definiert ist.

25. Verfahren nach einem der Ansprüche 20 bis 24, wobei die mRNA bei der Transfektion gemäß Schritt (b) mit mindestens einem kationischen oder polykationischen Agens komplexiert oder kondensiert vorliegt.

26. Verfahren nach Anspruch 25, wobei das kationische oder polykationische Agens aus der Gruppe, bestehend aus Protamin, Poly-L-Lysin, Poly-L-Arginin und Histonen, ausgewählt ist.

27. Verfahren nach einem der Ansprüche 20 bis 26, wobei die mRNA durch ein Verfahren hergestellt wird, das die folgenden Schritte umfasst:
(1) Herstellen einer cDNA-Bibliothek oder eines Teils davon aus Tumorgewebe oder aus mit einem pathogenen Agens infizierten Zellen eines Patienten,
(2) Herstellen einer Matrize für die *In vitro* -Transkription von RNA anhand der cDNA-Bibliothek oder eines Teils davon und
(3) *In vitro*-Transkribieren der Matrize.

28. Verfahren nach Anspruch 27, wobei der Teil der cDNA-Bibliothek des Tumorgewebes für die tumorspezifischen Antigene oder für die aufgrund der Infektion mit dem pathogenen Agens hochregulierten Genprodukte codiert.

29. Verfahren nach Anspruch 28, in welchem vor Schritt (1) die Sequenzen der tumorspezifischen Antigene oder die Sequenzen der aufgrund der Infektion mit dem pathogenen Agens hochregulierten Genprodukte ermittelt werden.

30. Verfahren nach Anspruch 29, wobei das Ermitteln der Sequenzen der tumorspezifischen Antigene oder der Sequenzen der durch die Infektion mit einem pathogenen Agens hochregulierten Genprodukte einen Abgleich mit einer cDNA-Bibliothek aus gesundem Gewebe bzw. aus nicht infizierten Zellen umfasst.

31. Verfahren nach Anspruch 30, wobei das Ermitteln der Sequenzen der tumorspezifischen Antigene oder der Sequenzen der aufgrund der Infektion mit einem pathogenen Agens hochregulierten Genprodukte eine Diagnose durch einen Mikroarray umfasst.

32. Pharmazeutische Zusammensetzung, erhältlich durch das Verfahren nach einem der Ansprüche 20 bis 31.

33. Verwendung von Blutzellen, die mit mindestens einer mRNA transfiziert sind, welche mindestens einen für mindestens ein Antigen codierenden Bereich umfasst, gegebenenfalls in Verbindung mit einem pharmazeutisch verträglichen Träger und/oder Vehikel, wobei die Blutzellen im wesentlichen reine oder angereicherte PBMCs sind und nicht mehr als 5% dendritische Zellen (DCs) enthalten, zur Herstellung eines Arzneimittels zur Stimulation einer Immunantwort gegen das oder die von der mRNA codierte(n) Antigen(e).

34. Verwendung nach Anspruch 33, wobei die Blutzellen nicht mehr als 2 % dendritische Zellen (DCs) enthalten.

35. Verwendung nach Anspruch 33 oder 34, wobei die Blutzellen autolog sind.

36. Verwendung nach einem der Ansprüche 33 bis 35, wobei die mRNA wie in einem der Ansprüche 4 bis 19 definiert ist.

37. Verwendung nach einem der Ansprüche 33 bis 36, wobei die Immunstimulation der Therapie von und/oder Prophylaxe gegen Krebs oder Infektionskrankheiten dient.
